(19) Europäisches Patentamt
European Patent Office
Office européen des brevets

(11) Veröffentlichungsnummer: **0 224 076**
**B1**

(12) # EUROPÄISCHE PATENTSCHRIFT

(45) Veröffentlichungstag der Patentschrift: **08.11.89**

(51) Int. Cl.⁴: **C07D 249/08**, C07D 233/60, A01N 43/50, A01N 43/653

(21) Anmeldenummer: **86115194.2**

(22) Anmeldetag: **03.11.86**

(54) **Azolyletherketone und -alkohole.**

(30) Priorität: **15.11.85 DE 3540523**

(43) Veröffentlichungstag der Anmeldung:
**03.06.87 Patentblatt 87/23**

(45) Bekanntmachung des Hinweises auf die Patenterteilung:
**08.11.89 Patentblatt 89/45**

(84) Benannte Vertragsstaaten:
**AT BE CH DE ES FR GB IT LI NL**

(56) Entgegenhaltungen:
**DE-A- 2 632 601**
**DE-A- 2 811 919**
**DE-A- 2 842 137**

**CHEMICAL ABSTRACTS, Band 93, Nr. 15, 13. Oktober 1980, Columbus, Ohio, USA NISHIYAMA, RYUZO; HAGA, TAKAHIRO; KOMYOJI, TERUMASA (ISHIHARA SANGYO KAISHA, LTD.) "Pyridyloxyalkylazoles" Seite 720, Spalte 2, Zusammenfassung Nr. 150 257y**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk(DE)**

(72) Erfinder: **Jäger, Gerhard, Dr., Paul-Klee-Strasse 68j, D-5090 Leverkusen 1(DE)**
Erfinder: **Jautelat, Manfred, Dr., Müllersbaum 28, D-5093 Burscheid(DE)**
Erfinder: **Krämer, Wolfgang, Dr., Rosenkranz 25, D-5093 Burscheid(DE)**
Erfinder: **Büchel, Karl Heinz, Prof. Dr., Dabringhausener Strasse 42, D-5093 Burscheid(DE)**
Erfinder: **Reinecke, Paul, Dr., Steinstrasse 8, D-5090 Leverkusen(DE)**
Erfinder: **Brandes, Wilhelm, Dr., Eichendorffstrasse 3, D-5653 Leichlingen(DE)**
Erfinder: **Hänssler, Gerd, Dr., Am Arenzberg 58a, D-5090 Leverkusen 3(DE)**
Erfinder: **Lürssen, Klaus, Dr., August-Kierspel-Strasse 151, D-5060 Bergisch Gladbach(DE)**

**EP 0 224 076 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Azolyletherketone und -alkohole, mehrere Verfahren zu ihrer Herstellung sowie ihre Verwendung als Fungizide und Pflanzenwachstumsregulatoren.

Es ist bereits bekannt, daß zahlreiche Azolyletherketone und -alkohole fungizide und pflanzenwachstumsregulierende Eigenschaften besitzen (vgl. DE- A 2 632 603, DE-A 2 333 355, DE-A 2 325 156, DE-A 2 324 010, DE-A 2 105 490, DE-A 2 842 137 und DE-A 2 632 601).

So lassen sich z.B. 4-Chlor-3,3-dimethyl-1-(2,4-dichlorphenoxy)-1-(1,2,4-triazol-1-yl)-butan-2-on, 3,3-Dimethyl-1-1-(4-fluorphenoxy)-1-(1,2,4-triazol-1-yl)-butan-ol, 3,3-Dimethyl-1-(2,4-dichlorphenoxy)-1-(1,2,4-triazol-1-yl)-butan-2-on, 3,3-Dimethyl-1-(4-fluorphenoxy)-1-(imidazol-1-yl)-butan-2-ol, 4-Chlor-1-(4-chlorphenoxy)-3,3-dimethyl-1-(1,2,4-triazol-1-yl)-butan-2-on, 3,3-Dimethyl-1-(2,4-dichlorphenoxy)-1-(1,2,4-triazol-1-yl)-butan-2-ol, 1-(4-Chlorphenoxy)-3,3-dimethyl-1-(triazol-1-yl)-butan-2-ol, 1-(4-(Biphenoxy)-4-chlor-3,3-dimethyl-1-(imidazol-1-yl)-butan-2-on und 1-(4-Biphenoxy)-3,3-dimethyl-1-(imidazol-1-yl)-butan-2-ol als Fungizide verwenden. Die Wirksamkeit dieser Stoffe ist jedoch vor allem bei niedrigen Aufwandmengen nicht immer befriedigend.

Ferner ist bereits bekannt geworden, daß Zinkethylen-1,2-bisdithiocarbamidat gut zur Bekämpfung von pilzlichen Pflanzenkrankheiten eingesetzt werden kann (vgl. Phytopathology 33, 1113 (1963). Die Verwendung dieses Stoffes ist aber dadurch beeinträchtigt, daß die Wirkung bei niedrigen Dosierungen nicht immer ausreichend ist.

Es wurden nun neue Azolyletherketone und -alkohole der Formel (I),

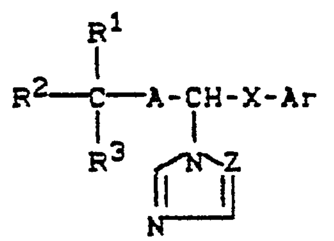

(I)

in welcher

$R^1$ für geradkettiges oder verzweigtes Alkyl mit 2 bis 4 Kohlenstoffatomen steht,
$R^2$ für geradkettiges oder verzweigtes Alkyl mit 2 bis 4 Kohlenstoffatomen steht,
$R^3$ für geradkettiges oder verzweigtes Alkyl mit 1 bis 4 Kohlenstoffatomen steht,
A für die Ketogruppe oder eine CH(OH)-Gruppe steht,
Z für ein Stickstoffatom oder die CH-Gruppe steht,
X für Sauerstoff steht und
Ar für Phenyl steht, das einfach bis dreifach, gleichartig oder verschieden substituiert sein kann durch Fluor, Chlor, Methyl und Phenyl,

oder

$R^1$ für Dichlormethyl steht,
$R^2$ für Methyl steht,
$R^3$ für Methyl steht,
A für die Ketogruppe oder eine CH(OH)-Gruppe steht,
Z für ein Stickstoffatom oder die CH-Gruppe steht,
X für Sauerstoff steht und
Ar für Phenyl steht, das einfach bis dreifach, gleichartig oder verschieden substituiert sein kann durch Fluor, Chlor, Methyl und Phenyl,

sowie deren Säureadditionssalze und Metallsalzkomplexe gefunden.

Die Verbindungen der Formel (I), in denen A für eine Ketogruppe steht, besitzen mindestens ein asymmetrisch substituiertes Kohlenstoffatom, und die Verbindungen der Formel (I), in denen A für die CH(OH)-Gruppe steht, besitzen mindestens zwei asymmetrisch substituierte Kohlenstoffatome. Die Verbindungen der Formel (I) können deshalb in verschiedenen optischen Isomerenformen vorliegen, die in unterschiedlichen Mengenverhältnissen anfallen können. Diejenigen Verbindungen der Formel (I), in denen $R^1$, $R^2$ und $R^3$ verschieden sind, enthalten ein zusätzliches asymmetrisch substituiertes Kohlenstoffatom, womit sich weitere optische Isomere ergeben. Die Erfindung betrifft sowohl Racemate als auch die einzelnen Isomeren und deren Gemische.

Weiterhin wurde gefunden, daß man neue Azolyletherketone und -alkohole der Formel (I) sowie deren Säureadditions-Salze und Metallsalz-Komplexe erhält, wenn man

a) Halogenetherketone der Formel (II)

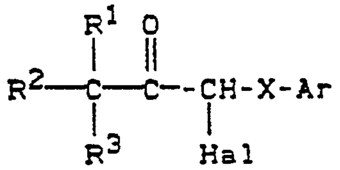

(II)

in welcher

$R^1$, $R^2$, $R^3$, X und Ar die oben angegebenen Bedeutungen haben und

Hal für Chlor oder Brom steht, mit 1,2,4-Triazol oder Imidazol, gegebenenfalls in Gegenwart eines Säurebindemittels und gegebenenfalls in Gegenwart eines Verdünnungsmittels, umsetzt und gegebenenfalls

b) die nach Verfahren (a) erhaltenen Azolyletherketone der Formel

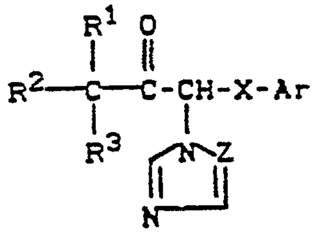

(Ia)

in welcher

$R^1$, $R^2$, $R^3$, Z, X und Ar die oben angegebenen Bedeutungen haben, gegebenenfalls in Gegenwart eines Verdünnungsmittels reduziert,

und gegebenenfalls anschließend eine Säure oder ein Metallsalz addiert.

Schließlich wurde gefunden, daß die neuen Azolyletherketone und -alkohole der Formel (I) sowie deren Säureadditionssalze und Metallsalzkomplexe sehr gute fungizide und pflanzenwuchsregulierende Eigenschaften aufweisen.

Überraschenderweise zeigen die erfindungsgemäßen Stoffe eine deutlich bessere fungizide und pflanzenwuchsregulierende Wirkung als die aus dem Stand der Technik bekannten Substanzen 4-Chlor-3,3-dimethyl (1-(2,4-dichlorphenoxy)-1-(1,2,4-triazol-1-yl)-butan-2-on, 3,3-Dimethyl-1-(4-fluorphenoxy)-1-(1,2,4-triazol-1-yl)-butan-2-ol, 3,3-Dimethyl-1-(2,4-dichlorphenoxy)-1-(1,2,4-triazol-1-yl)-butan-2-on, 3,3-Dimethyl-1-(4-fluorphenoxy)-1-(imidazol-1-yl)-butan-ol, 4-Chlor-1-(4-chlorphenoxy)-3,3-dimethyl-1-(1,2,4-triazol-1-yl)-butan-2-on, 3,3-Dimethyl-1-(2,4-dichlorphenoxy)-1-(1,2,4-triazol-1-yl)-butan-2-ol, 1-(4-Chlorphenoxy)-3,3-dimethyl-1-(triazol-1-yl)-butan-2-ol, 1-(4-Biphenoxy)-4-chlor-3,3-dimethyl-1-(imidazol-1-yl)-butan-2-on und 1-(4-Biphenoxy)-3,3-dimethyl-1-(imidazol-1-yl)-butan-2-ol, welche konstitutionell und wirkungsmäßig naheliegende Verbindungen sind. Außerdem besitzen die erfindungsgemäßen Stoffe auch eine bessere fungizide Wirkung als das aus dem Stand der Technik vorbekannte Zinkethylen-1,2-bis-dithiocarbamidat, welches eine wirkungsgemäß naheliegende Verbindung ist.

Die erfindungsgemäßen Azolyletherketone und -alkohole sind durch die Formel (I) eindeutig definiert.

Bevorzugte Säureadditions-Salze sind Additionsprodukte aus Säuren und denjenigen Azolyletherketonen und -alkoholen der Formel (I), in denen die Substituenten $R^1$, $R^2$, $R^3$, A, Z, X und Ar die Bedeutungen haben, die bereits für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Bevorzugte Metallsalz-komplexe sind Additionsprodukte aus Salzen von Metallen der II. bis IV. Haupt- und der I. und II. sowie IV. bis VIII. Nebengruppe des Periodensystems der Elemente und denjenigen substituierten Azolyletherketonen und -alkoholen der Formel (I), in denen die Substituenten $R^1$, $R^2$, $R^3$, A, X, Z und Ar die Bedeutungen haben, die bereits für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Verbindungen der Formel (I) genannt:

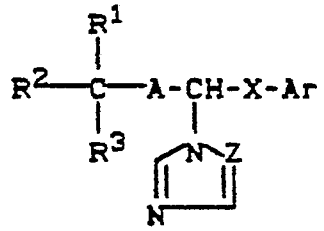

(I)

| $R^1$ | $R^2$ | $R^3$ | A | Z | X | Ar |
|---|---|---|---|---|---|---|
| $CHCl_2$ | $CH_3$ | $CH_3$ | CO | N | O | 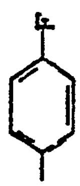 |
| $CHCl_2$ | $CH_3$ | $CH_3$ | CO | CH | O | 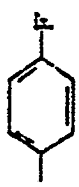 |
| $CHCl_2$ | $CH_3$ | $CH_3$ | CHOH | N | O | 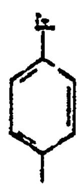 |
| $CHCl_2$ | $CH_3$ | $CH_3$ | CHOH | CH | O | 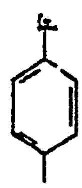 |
| $CHCl_2$ | $CH_3$ | $CH_3$ | CO | N | O | 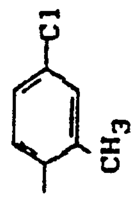 |
| $CHCl_2$ | $CH_3$ | $CH_3$ | CHOH | N | O | 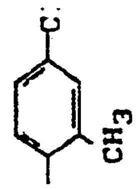 |
| n-$C_4H_9$ | $C_2H_5$ | $CH_3$ | CO | N | O | 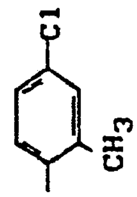 |
| n-$C_4H_9$ | $C_2H_5$ | $CH_3$ | CO | CH | O | 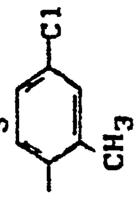 |

| $R^1$ | $R^2$ | $R^3$ | A | Z | X | Ar |
|---|---|---|---|---|---|---|
| $C_2H_5$ | $C_2H_5$ | $CH_3$ | CO | N | O | 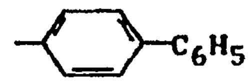 |
| $C_2H_5$ | $C_2H_5$ | $CH_3$ | CO | CH | O | 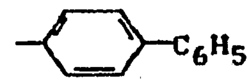 |
| $C_2H_5$ | $C_2H_5$ | $CH_3$ | CHOH | N | O | 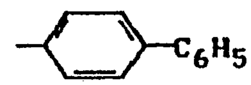 |
| $C_2H_5$ | $C_2H_5$ | $CH_3$ | CHOH | CH | O | 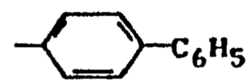 |
| $C_2H_5$ | $C_2H_5$ | $CH_3$ | CO | N | O | 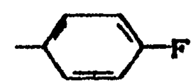 |
| $C_2H_5$ | $C_2H_5$ | $CH_3$ | CO | CH | O | 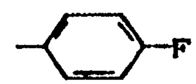 |
| $C_2H_5$ | $C_2H_5$ | $CH_3$ | CHOH | N | O | 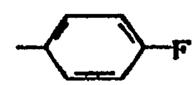 |
| $C_2H_5$ | $C_2H_5$ | $CH_3$ | CO | CH | O | 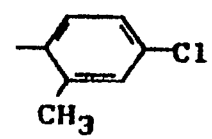 |

| $R^1$ | $R^2$ | $R^3$ | A | Z | X | Ar |
|---|---|---|---|---|---|---|
| $C_2H_5$ | $C_2H_5$ | $CH_3$ | CO | N | O | 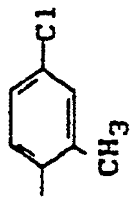 |
| $C_2H_5$ | $C_2H_5$ | $C_2H_5$ | CO | N | O | 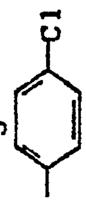 |
| $C_2H_5$ | $C_2H_5$ | $C_2H_5$ | CO | CH | O | 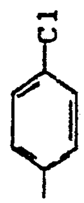 |
| $C_2H_5$ | $C_2H_5$ | $C_2H_5$ | CHOH | N | O | 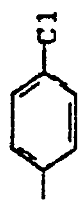 |
| $C_2H_5$ | $C_2H_5$ | $C_2H_5$ | CHOH | CH | O | 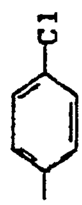 |

Verwendet man beispielsweise 1-Brom-1-(2,4-dichlorphenoxy)-3-ethyl-3-methyl-pentan-2-on und 1,2,4-Triazol als Ausgangsstoffe, so kann der Verlauf des erfindungsgemäßen Verfahrens (a) durch das folgende Formelschema wiedergegeben werden:

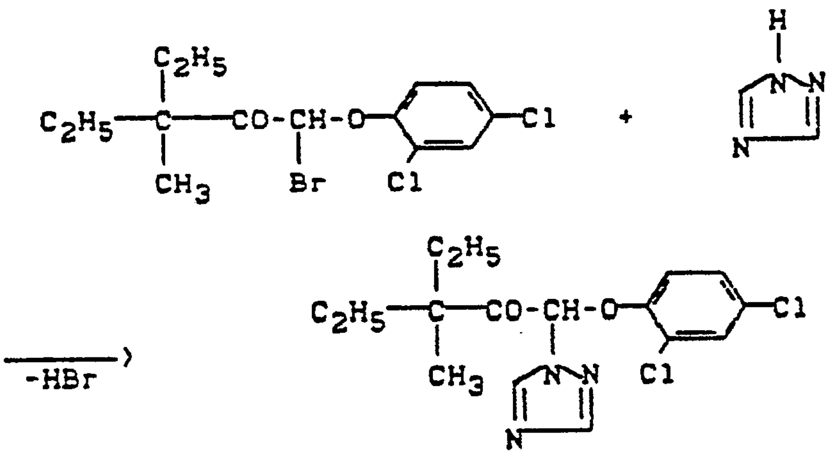


Verwendet man beispielsweise 4,4-Dichlor-1-(2,4-dichlorphenoxy)-3,3-dimethyl-1-(1,2,4-triazol-1-yl)-butan-2-on als Ausgangsstoff und Natriumborhydrid als Reduktionsmittel, so kann der Verlauf des erfindungsgemäßen Verfahrens (b) durch das folgende Formelschema wiedergegeben werden:

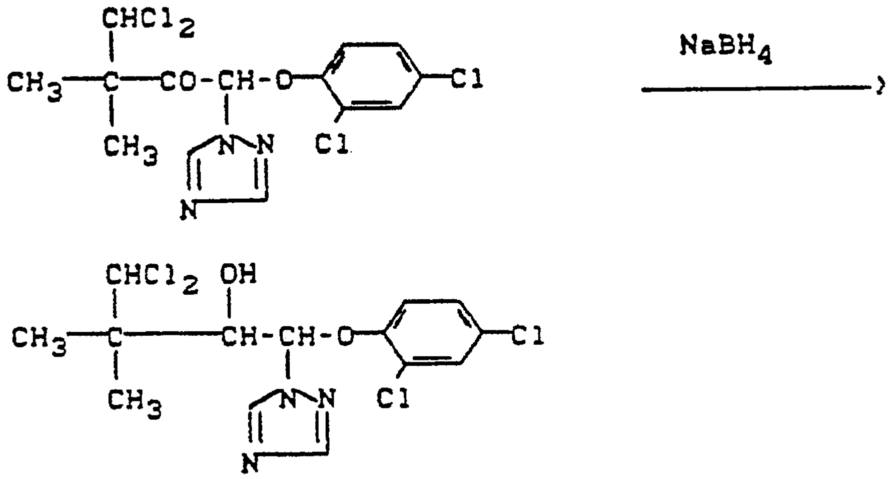


Die für das erfindungsgemäße Verfahren (a) als Ausgangsstoffe zu verwendenden Halogenetherketone sind durch die Formel (II) allgemein definiert. In dieser Formel stehen $R^1$, $R^2$, $R^3$, X und Ar für die Reste, die bereits im Zusammenhang mit der Beschreibung der erfindungsgemäßen Stoffe der Formel (I) für diese Substituenten genannt wurden. Hal steht für Chlor oder Brom.

Die Halogenetherketone der Formel (II) sind noch nicht bekannt; sie können jedoch in allgemein bekannter Art und Weise erhalten werden, indem man z.B. Etherketone der Formel (III)

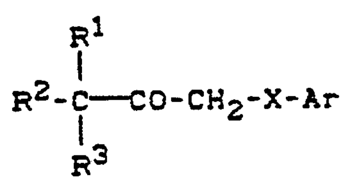 (III)

in welcher

$R^1$, $R^2$, $R^3$, X und Ar die oben angegebenen Bedeutungen haben,

in Gegenwart eines Lösungsmittels wie z.B. Dichlormethan, Trichlormethan, Tetrachlormethan, Toluol oder Chlorbenzol, mit einem Halogenierungsmittel wie z.B. Brom, Sulfurylchlorid oder N-Bromsuccinimid, bei Temperaturen zwischen 0 und 100°C, vorzugsweise zwischen 10 und 80°C, halogeniert. Die entstehenden Halogenetherketone der Formel (II) können gegebenenfalls ohne Isolierung weiter umgesetzt werden.

Die Etherketone der Formel (III) sind ebenfalls noch nicht bekannt; sie können jedoch in allgemein bekannter Art und Weise erhalten werden, indem man Halogenketone der Formel (IV)

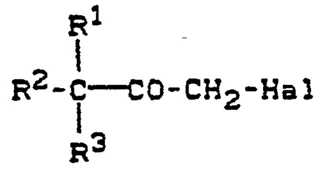 (IV)

in welcher
$R^1$, $R^2$, $R^3$ und Hal die oben angegebenen Bedeutungen haben,
mit Phenolen der Formel (V)

AR–X–H (V)

in welcher
Ar und X die oben angegebenen Bedeutungen haben,
in Gegenwart einer starken Base wie z.B. Kalium- oder Natriumcarbonat oder Triethylamin, und in Gegenwart eines inerten organischen Lösungsmittels wie z.B. N,N-Dimethylformamid, Aceton, Methylisopropylketon oder Acetonitril, bei Temperaturen zwischen 50 und 150°C, vorzugsweise zwischen 80 und 120°C, umsetzt.

Die Phenole der Formel (V) sind allgemein bekannte Verbindungen der organischen Chemie.

Die Halogenketone der Formel (IV) sind noch nicht bekannt; sie können erhalten werden, indem man z.B. Verbindungen der allgemeinen Formel (VI),

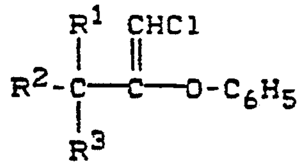 (VI)

in welcher
$R^1$, $R^2$ und $R^3$ die oben angegebenen Bedeutungen haben,
in Gegenwart einer Säure, wie z.B. einer Mineralsäure vorzugsweise Schwefelsäure oder Salzsäure, und/oder einer organischen Säure, wie Ameisensäure, Trifluoressigsäure, Oxalsäure, p-Toluolsulfonsäure oder Methansulfonsäure, die auch in wässriger Verdünnung vorliegen kann, bei Temperaturen zwischen 20 und 150°C, vorzugsweise zwischen 40 und 100°C, in Gegenwart eines Verdünnungsmittels wie z.B. Methanol, Ethanol, Aceton, Dichlormethan oder Dioxan, hydrolysiert; (vgl. DE-A 3 049 461 und Herstellungsbeispiele).

Die Verbindungen der Formel (VI) sind noch nicht bekannt; sie können erhalten werden, indem man z.B. 1,1-Dichloralkene der allgemeinen Formel (VII),

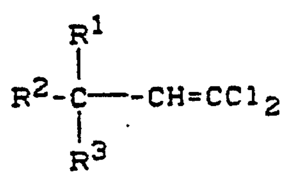 (VII)

in welcher
$R^1$, $R^2$ und $R^3$ die oben angegebenen Bedeutungen haben,
mit Phenolaten wie z.B. Natrium- oder Kaliumphenolat in Gegenwart eines Verdünnungsmittels, wie z.B. Dimethylformamid, N-Methylpyrrolidon, Dimethylsulfoxid, 1,1-Dimethyl-2-imidazolidon oder Tetramethylharnstoff, bei Temperaturen zwischen 50 und 250°C umsetzt (vgl. DE-A 3 049 461).

Die 1,1-Dichloralkene der Formel (VII) sind noch nicht bekannt; sie können jedoch in allgemein bekannter Art und Weise erhalten werden, indem man z.B. Chlorverbindungen der Formel (VIII),

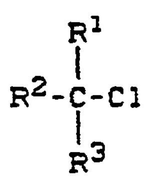 (VIII)

in welcher
$R^1$, $R^2$ und $R^3$ die oben angegebenen Bedeutungen haben,

mit 1,1-Dichlorethen in Gegenwart von Metallhalogeniden wie z.B. Eisen(III)-chlorid, Aluminium(III)-chlorid oder Wismut(III)-chlorid bei Temperaturen zwischen −78 und 50°C umsetzt.

Die Chlorverbindungen der Formel (VIII) sind allgemein bekannte Verbindungen der organischen Chemie.

Das für das erfindungsgemäße Verfahren (a) als Ausgangsstoff zu verwendende 1,2,4-Triazol sowie das als Ausgangsstoff zu verwendende Imidazol sind allgemein bekannte Verbindungen der organischen Chemie.

Die für die Durchführung des erfindungsgemäßen Verfahrens (b) als Ausgangsstoffe zu verwendenden Azolyletherketone der Formel (Ia) sind erfindungsgemäße Verbindungen und können nach dem erfindungsgemäßen Verfahren (a) hergestellt werden.

Das erfindungsgemäße Verfahren (a) wird vorzugsweise in Gegenwart eines inerten organischen Lösungsmittels durchgeführt. Vorzugsweise verwendbar sind Nitrile, wie Propionitril, insbesondere Acetonitril; Formamide, wie insbesondere Dimethylformamid; Ketone, wie Diethylketon, Aceton und Methylethylketon; Alkohole, wie Ethanol und Isopropanol; Ether, wie Tetrahydrofuran und Dioxan; aromatische Kohlenwasserstoffe wie Benzol und Toluol, und halogenierte Kohlenwasserstoffe wie Dichlormethan.

Die erfindungsgemäße Umsetzung gemäß Verfahren (a) wird vorzugsweise in Gegenwart eines Säurebinders vorgenommen. Man kann alle üblicherweise verwendbaren anorganischen oder organischen Säurebinder zugeben, wie Akalicarbonate, beispielsweise Natriumcarbonat, Kaliumcarbonat und Natriumhydrogencarbonat, oder niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan. Vorzugsweise verwendet man einen entsprechenden Überschuß an 1,2,4-Triazol bzw. Imidazol.

Die Reaktionstemperaturen können beim Verfahren (a) in einem größeren Bereich variiert werden. Im allgemeinen arbeitet man zwischen 20 und 150°C, vorzugsweise zwischen 60 und 120°C. Bei Anwesenheit eines Lösungsmittels wird zweckmäßigerweise beim Siedepunkt des jeweiligen Lösungsmittels gearbeitet.

Bei der Durchführung des erfindungsgemäßen Verfahrens (a) setzt man auf 1 Mol der Verbindungen der Formel (II) vorzugsweise 1 bis 2 Mol 1,2,4-Triazol bzw. Imidazol und 1 bis 2 Mol Säurebinder ein. Zur Isolierung der Verbindungen der Formel (I) wird im allgemeinen das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt. Außerdem sind auch andere Aufarbeitungsmethoden durchführbar.

Die erfindungsgemäße Reduktion gemäß Verfahren (b) erfolgt in üblicher Weise, z.B. durch Umsetzung mit komplexen Hydriden, gegebenenfalls in Gegenwart eines Verdünnungsmittels oder durch Umsetzung mit Aluminiumisopropylat in Gegenwart eines Verdünnungsmittels.

Arbeitet man mit komplexen Hydriden, so kommen als Verdünnungsmittel für die erfindungsgemäße Umsetzung polare organische Lösungsmittel in Frage. Hierzu gehören vorzugsweise Alkohole wie Methanol, Ethanol, Butanol, Isopropanol und Ether wie Diethylether oder Tetrahydrofuran. Die Reaktion wird im allgemeinen bei −10 bis + 30°C, vorzugsweise bei 0 bis 20°C durchgeführt. Hierzu setzt man auf 1 Mol des Ketons der Formel (Ia) etwa 1 Mol eines komplexen Hydrids, wie Natriumborhydrid oder Lithiumalanat, ein. Zur Isolierung der reduzierten Verbindungen der Formel (I) wird der Rückstand im allgemeinen in verdünnter Salzsäure aufgenommen, anschließend alkalisch gestellt und mit einem organischen Lösungsmittel extrahiert. Die weitere Aufarbeitung erfolgt in üblicher Art und Weise.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol infrage. Die Reaktionstemperaturen können wiederum in einem größeren Bereich variiert werden; im allgemeinen arbeitet man zwischen 20 und 120°C, vorzugsweise zwischen 50 und 100°C. Zur Durchführung der Reaktion setzt man auf 1 Mol eines Ketons der Formel (Ia) etwa 0,3 bis 3 Mol Aluminiumisopropylat ein. Zur Isolierung der reduzierten Verbindungen der Formel (I) wird im allgemeinen das überschüssige Lösungsmittel im Vakuum entfernt, und die entstandenen Aluminium-Verbindungen werden mit verdünnter wäßriger Schwefelsäure oder Natronlauge zerstetzt. Die weitere Aufarbeitung erfolgt in üblicher Weise.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der allgemeinen Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der allgemeinen Formel (I) kommen vorzugsweise diejenigen Salze von Metallen in Frage, die bereits weiter oben beschrieben wurden.

Die Metallsalz-Komplexe von Verbindungen der allgemeinen Formel (I) können in einfacher Weise nach üblichen Verfahren erhalten werden, so z.B. durch Lösen des Metallsalzes in Alkohol, z.B.

Ethanol, und Hinzufügen zu Verbindungen der allgemeinen Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder
Pseudoperonospora cubense;
Plasmopara-Arten, wie Plasmopara viticola;
Peronospora-Arten, wie Peronospora pisi oder
P. brassicae;
Eryisiphe-Arten, wie Erysiphe graminis;
Sphaerotheca-Arten, wie Sphaerotheca fuliginea;
Podosphaera-Arten-wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;
Puccinia-Arten, wie Puccinia recondita;
Tilletia-Arten, wie Tilletia caries;
Ustilago-Arten, wie Ustilago nuda oder
Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Puccinia-Arten wie Puccinia recondita und Leptosphaeria-Arten wie Leptosphaeria nodorum an Weizen; ferner zur Bekämpfung von Sphaerotheca-Arten wie Sphaerotheca fuliginea an der Gurke, Venturia-Arten wie Venturia inequalis an Apfel, Uromyces-Arten wie Uromyces appendiculatus an der Buschbohne sowie auch zur Bekämpfung von Pyricularia oryzae an Reis eingesetzt werden. Ferner eignen sich die erfindungsgemäßen Wirkstoffe sehr gut zur Bekämpfung von Mehltau sowie Cochiobolus sativus und Pyrenophora teres an Getreide.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums

der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, da z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von

Wachsturmsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formlierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Durck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsufloxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetet, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoffe, vorzugsweise zwischen 0,5 und 90%.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsgulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-% vorzugsweise zwischen 0,5 und 0,001%. Bei der Saatgutbehandlung werden im allgemeinen Wirkstoffmengen von 0,001 bis 50 g je Kiligramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02%, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Herstellung und Verwendung der erfindungsgemäßen Wirkstoffe gehen aus den folgenden Beispielen hervor.

Herstellungsbeispiele

Beispiel 1

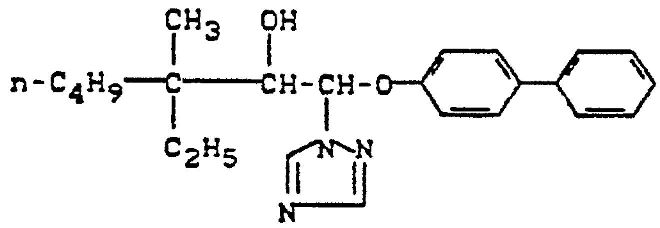

(Verfahren b)

Zu einer Lösung von 7 g (0,02 Mol) 1-(4-Phenylphenoxy)-3-ethyl-3-methyl-1-(1,2,4-triazol-1-yl)-heptan-2-on in 70 ml Methanol werden bei 20–30°C portionsweise unter Rühren 1,5 g (0,04 Mol) Natriumboranat eingetragen. Nach 2 Stunden wird die Lösung durch Zugabe von Essigsäure auf pH 6 eingestellt und eingedampft. Der Rückstand wird in 150 ml Dichlormethan aufgenommen, die Lösung dreimal mit je 250 ml Wasser gewaschen, über Natriumsulfat getrocknet und im Vakuum eingedampft.

Durch Anreiben des Rückstandes mit wenig Diethylether erhält man 3,9 g (56% der Theorie) 1-(4-Phenylphenoxy)-3-ethyl-3-methyl-1-(1,2,4-triazol-1-yl)-heptan-2-ol (Diastereomerengemisch) vom Schmelzpunkt 120–122°C.

Beispiel 2:

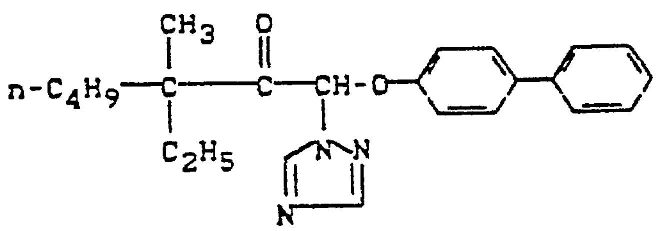

(Verfahren a)

14,5 g (0,21 Mol) 1,2,4-Triazol werden mit 29 g (0,07 Mol) 1-Brom-1-(4-phenylphenoxy)-3-ethyl-3-methyl-heptan-2-on in 180 ml Acetonitril 10 Stunden zum Sieden erhitzt. Das Reaktionsgemisch wird unter vermindertem Druck eingedampft, der Rückstand in 300 ml Dichlormethan aufgenommen und dreimal mit je 400 ml Wasser gewaschen. Nach dem Trocknen der organischen Phase über Natriumsulfat wird das Lösungsmittel im Vakuum abdestilliert und der Rückstand mit Trichlormethan als Laufmittel an Kieselgel chromatographiert.

Man erhält 12 g (44% der Theorie) and 1-(4-Phenylphenoxy)-3-ethyl-3-methyl-1-(1,2,4-triazol-1-yl)-heptan-2-on vom Schmelzpunkt 67–68°C und 3 g (11% der Theorie) des isomeren 1-(4-Phenyl-phenoxy)-3-ethyl-3-methyl-1-(1,2,4-triazol-4-yl)-heptan-2-on vom Schmelzpunkt 154–155°C.

Herstellen des Ausgangsproduktes:

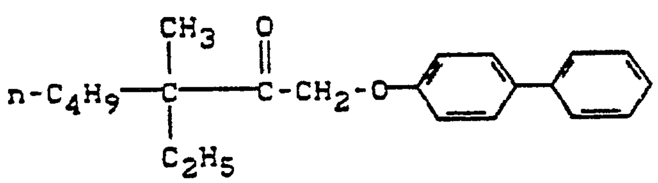

Zu einer Lösung von 56 g (0,172 Mol) 1-(4-Phenylphenoxy)-3-ethyl-3-methyl-heptan-2-on in 200 ml Dichlormethan werden bei 20–30°C unter Rühren 27,5 g (0,172 Mol) Brom zugetropft. Nach 15 Minuten werden 100 ml einer wäßrigen Natriumbisulfit-Lösung zugesetzt. Die organische Phase wird abgetrennt, dreimal mit je 400 ml Wasser gewaschen, über Natriumsulfat getrocknet und unter vermindertem Druck eingedampft.

Es werden 59 g (85% der Theorie) rohes 1-Brom-1-(4-phenylphenoxy)-3-ethyl-3-methyl-heptan-2-on als bräunlich, viskoses Öl erhalten.

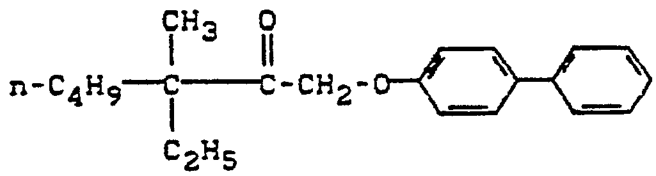

44,2 g (0,26 Mol) 4-Hydroxy-biphenyl werden mit 35,9 g (0,26 Mol) Kaliumcarbonat und 50 g (0,26 Mol) 1-Chlor-3-ethyl-3-methyl-heptan-2-on in 280 ml Aceton 10 Stunden zum Sieden erhitzt. Nach dem Abkühlen auf Raumtemperatur wird filtriert, das Filtrat im Vakuum eingedampft und der Rückstand 1 Stunde bei 0,1 mbar auf 200°C erhitzt.

Man erhält 56 g (66% der Theorie) an 1-(4-Phenylphenoxy)-3-ethyl-3-methyl-heptan-2-on als schwach gelbliches hochviskoses Öl.

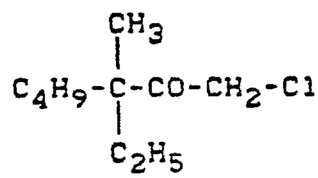

895 g (3,36 Mol) 1-Chlor-3-ethyl-3-methyl-2-phenoxy-1-hepten werden in 1 l Ethanol mit 500 ml konz. Salzsäure 5 Stunden unter Rückfluß erhitzt. Man verdünnt mit Methylenchlorid, schüttelt zunächst mit Wasser, dann mehrfach mit 2 n wäßriger Natronlauge aus.

Nach Trocknen und Einrotieren erhält man 865 g Rohprodukt, das bei der Destillation (Kp $_{0,3}$ 65°C) 520 g (81% der Theorie) 1-Chlor-3-ethyl-3-methyl-heptan-2-on liefert.

NMR ($CDCl_3$): δ 0,7–1,7 (m, 17 H), 4,3 (s, 2 H)

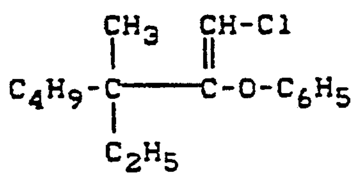

626 g (3 Mol) 1,1-Dichlor-3-ethyl-3-methyl-1-hepten und 696 g (6 Mol) Natriumphenolat werden in 3 l Dimethylformamid 16 Stunden unter Rückfluß erhitzt. Es wird mit Methylenchlorid verdünnt und mit 2 n wäßriger Natronlauge mehrfach ausgeschüttelt. Nach Trocknen und Einrotieren bleiben 666 g Rohprodukt zurück. Durch Destillation bei Kp $_{0,1}$ 100–110°C gewinnt man 523 g (65% der Theorie) 1-Chlor-3-ethyl-3-methyl-2-phenoxy-hept-1-en.

NMR ($CDCl_3$): δ 0,7–1,6 (m, 17 H), 5,8 und 5,85 (2 s, 1 H), 6,8–7,4 (m, 5 H).

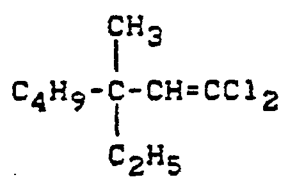

1235 g (12,7 Mol) 1,1-Dichlorethen und 630 g (4,23 Mol) 3-Chlor-3-methylheptan werden bei 20°C vorgelegt. 85 g wasserfreies Eisen(III)-chlorid werden portionsweise unter Kühlen zugegeben. Anschließend rührt man 2 Stunden bei 30°C nach. Der Ansatz wird auf Eis und verdünnte wäßrige Salzsäure gegossen. Die organische Phase wird abgetrennt, getrocknet und fraktioniert destilliert.

Bei Kp $_{6,1}$ 50–60°C isoliert man 627 g (71% der Theorie) 1,1-Dichlor-3-ethyl-3-methyl-hept-1-en.

NMR ($CDCl_3$): δ 0,7–1,8 (m, 17 H), 5,75 und 5,85 (2 s, 1 H)

Beispiel 3:

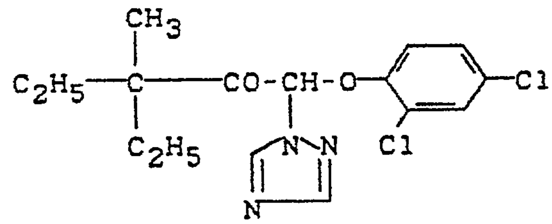

(Verfahren a)

24,2 g (0,351 Mol) 1,2,4-Triazol und 43 g (0,117 Mol) 1-Brom 1-(2,4-dichlorphenoxy)-3-ethyl-3-methyl-pentan-2-on in 230 ml Acetonitril werden 10 Stunden zum Sieden erhitzt. Man verdünnt mit 300 ml Dichlormethan, wäscht die Lösung dreimal mit je 400 ml Wasser und destilliert nach dem Trocknen über Natriumsulfat das Lösungsmittel unter vermindertem Druck ab. Durch fraktionierte Kristallisation des Rückstandes aus Diethylether/Petrolether (1 : 1) werden erhalten:

24 g (58% der Theorie) 1-(2,4-Dichlorphenoxy)-3-ethyl-3-methyl-1-(1,2,4-triazol-1-yl)-pentan-2-on vom Schmelzpunkt 68–69° C und 7,3 g (17,5% der Theorie) des isomeren 1-(2,4-Dichlorphenoxy)-3-ethyl-3-methyl-1-(1,2,4-triazol-1-yl)-pentan-2-on vom Schmelzpunkt 154–155°C.

Herstellung des Vorproduktes:

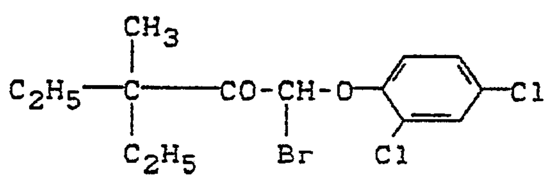

Zu einer Lösung von 75,7 g (0,238 Mol) 1-(2,4-Dichlor-phenoxy)-3-ethyl-3-methyl-pentan-2-on in 300 ml Dichlormethan werden unter Rühren 38,4 g (0,24 Mol) Brom zugetropft. Nach 15 Minuten werden 100 ml wäßrige Natriumbisulfit-Lösung zugesetzt. Die organische Phase wird abgetrennt, dreimal mit je 400 ml Wasser gewaschen und nach dem Trocknen über Natriumsulfat im Vakuum eingedampft.

Man erhält 87 g (99% der Theorie) rohes 1-Brom-1-(2,4-dichlorphenoxy)-3-ethyl-3-methyl-pentan-2-on als bräunliche Flüssigkeit, die ohne weitere Reinigung weiter umgesetzt wird.

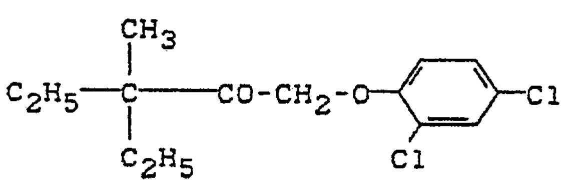

Zu einem Gemisch von 38,6 g (0,28 Mol) Kaliumcarbonat und 45,6 g (0,28 Mol) 2,4-Dichlorphenol in 200 ml N,N-Dimethylformamid werden in der Siedehitze 50 g (0,28 Mol) 1-Chlor-3-ethyl-3-methyl-pentan-2-on, gelöst in 30 ml N,N-Dimethylformamid, zugegeben. Man erhitzt 10 Stunden zum Sieden, kühlt auf Raumtemperatur, filtriert und destilliert das Lösungsmittel unter vermindertem Druck ab.

Durch Destillation des Rückstandes erhält man 75,7 g (93,5% der Theorie) an 1-(2,4-Dichlorphenoxy)-3-ethyl-3-methyl-pentan-2-on als farblose Flüssigkeit vom Siedepunkt 141–144°C/0,15 mm.

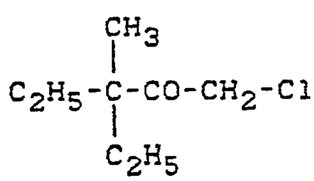

1522 g (6,38 Mol) 1-Chlor-3-ethyl-3-methyl-2-phenoxy-1-pent-1-en werden in 3,4 l Ameisensäure unter Zusatz von konz. Salzsäure 5 Stunden bei 60°C gerührt. Das Gemisch wird mit Methylenchlorid verdünnt, dann mit Wasser gewaschen und danach mehrfach mit 2 n wäßriger Natronlauge ausgeschüttelt. Nach Trocknen und Abziehen des Solvens bleiben 927 g Rohprodukt zurück. Die Destillation bei Kp $_{0,2}$

62–70°C liefert 878 g (85% der Theorie) 1-Chlor-3-ethyl-3-methyl-pentan-2-on.
NMR ($CDCl_3$): δ 0,8 (t, 6 H), 1,15 (s, 3 H), 1,6 (q, 4 H), 4,3 (s, 2 H).

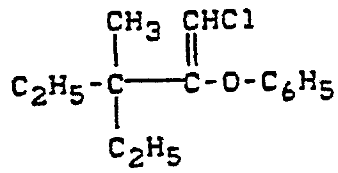

1786 g (8,23 Mol) 3-Ethyl-3-methyl-1,1,1-trichlorpentan werden zu einer Mischung aus 2030 g (17,5 Mol) Natriumphenolat und 1173 g (8,5 Mol) Kaliumcarbonat in 4 l N-Methylpyrrolidon bei 195–200°C getropft. Das Gemisch wird 8 Stunden bei 200°C nachgerührt. Nach Verdünnen mit Methylenchlorid wird mit Wasser und 2 n wäßriger Natronlauge mehrfach ausgeschüttelt.

Durch Destillation des Rohprodukts (2470 g) erhält man 1507 g (77% der Theorie) 1-Chlor-3-ethyl-3-methyl-2-phenoxy-pent-1-en.
NMR ($CDCl_3$): δ 0,7–1,7 (m, 13H), 5,8 (s, 1H), 6,8–7,5 (m, 5 H).

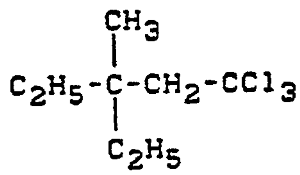

2328 g (24 Mol) 1,1-Dichlorethen werden bei –75°C vorgelegt. Es werden 80 g pulverisiertes Aluminiumchlorid zugegeben, und im Verlauf von 4 Stunden werden 964 g (8 Mol) 3-Chlor-3-methylpentan zugetropft. Man rührt 2 Stunden bei –70°C nach und gießt den Ansatz auf Eis und verdünnte Salzsäure. Nach Extraktion mit Methylenchlorid gewinnt man 1659 g Rohprodukt, das durch Destillation bei $Kp_{20}$ 95–110°C gereinigt wird.

Man erhält 1495 g (86% der Theorie) 3-Ethyl-3-methyl-1,1,1-trichlorpentan.
NMR ($CDCl_3$): δ 0,8 (t, 6 H), 1,15 (s, 3 H), 1,55 (q, 4 H), 2,8 (s, 2 H).

Beispiel 4:

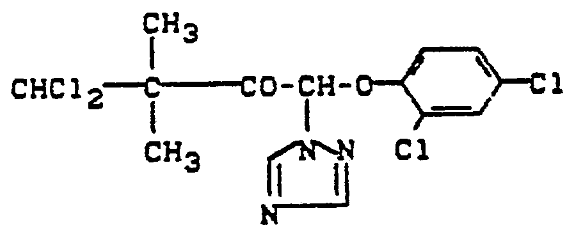

(Verfahren a)

Zu einer siedenden Lösung von 13,9 g (0,2 Mol) 1,2,4-Triazol in 150 ml Acetonitril werden 27,8 g (0,067 Mol) 1-Brom-4,4-dichlor-1-1(2,4-dichlorphenoxy)-3,3-dimethyl-butan-2-on, gelöst in 30 ml Acetonitril, zugegeben. Die Lösung wird acht Stunden zum Sieden erhitzt, auf 20°C gekühlt und mit 300 ml Dichlormethan verdünnt. Man wäscht anschließend dreimal mit je 300 ml Wasser, trocknet die organische Phase über Natriumsulfat, engt im Vakuum ein und filtriert den Rückstand über Kieselgel mit Trichlormethan als Elutionsmittel.

Man erhält 13 g (49% der Theorie) 4,4-Dichlor-1-(2,4-dichlorphenoxy)-3,3-dimethyl-1-(1,2,4-triazol-1-yl)-butan-2-on als farblose Kristalle vom Schmelzpunkt 120–121°C.

Durch weiteres Eluieren mit Essigsäureethylester werden noch 5 g (19% der Theorie) des isomeren 4,4-Dichlor-1-(2,4-dichlorphenoxy)-3,3-dimethyl-1-(1,2,4-triazol-1-yl)-butan-2-ons in Form farbloser Kristalle vom Schmelzpunkt 177–178°C erhalten.

Beispiel 5:

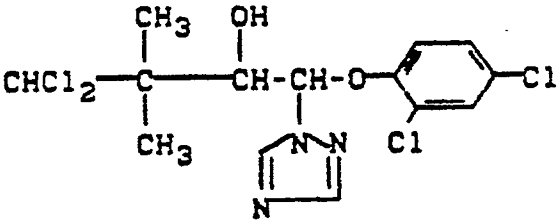

(Verfahren b)

Eine Lösung von 8 g (0,02 Mol) 4,4-Dichlor-1-(2,4-dichlorphenoxy)-3,3-dimethyl-1-(1,2,4-triazol-1-yl)-butan-2-on in 80 ml Methanol wird bei 20–30°C unter Rühren portionsweise mit 1,5 g (0,04 Mol) Natriumboranat versetzt. Nach zwei Stunden wird durch Zugabe von Essigsäure auf pH 6 eingestellt. Die Lösung wird unter vermindertem Druck eingedampft, der Rückstand in 200 ml Dichlormethan aufgenommen, dreimal mit je 400 ml Wasser gewaschen und über Natriumsulfat getrocknet. Nach dem Eindampfen der Lösung und Entfernen von Restlösungsmittel durch zweistündiges Erwärmen des Rückstandes auf 50°C bei 0,01 bar erhält man 6 g (75% der Theorie) 4,4-Dichlor-1-(2,4-dichlorphenoxy)-3,3-dimethyl-1-(1,2,4-triazol-1-yl)-butan-2-ol als 3 : 1 Gemisch der beiden Diastereomeren in Form eines hochviskosen Öls.

Beispiel 6:

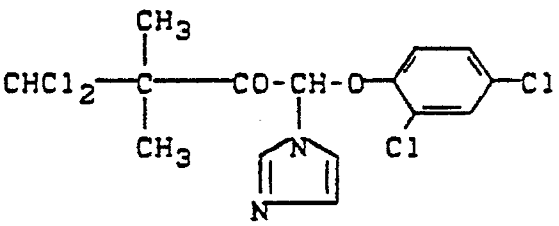

(Verfahren a)

10,2 g (0,15 Mol) Imidazol und 24 g (0,05 Mol) 1-Brom-4,4-dichlor-1-(2,4-dichlorphenoxy)-3,3-dimethyl-butan-2-on in 230 ml Acetonitril werden acht Stunden zum Sieden erhitzt. Nach dem Abkühlen auf Raumtemperatur wird mit 200 ml Dichlormethan verdünnt, und die Lösung wird dreimal mit je 400 ml Wasser gewaschen. Nach dem Trocknen der organischen Phase über Natriumsulfat wird im Vakuum eingedampft und der Rückstand über Kieselgel mit Trichlormethan als Fließmittel filtriert.

Man erhält 17 g (86% der Theorie) 4,4-Dichlor-1-(2,4-dichlorphenoxy)-3,3-dimethyl-1-(imidazol-1-yl)-butan-2-on als farblose Kristalle vom Schmelzpunkt 125–126°C.

In entsprechender Weise und analog den angegebenen Verfahren werden die Verbindungen der allgemeinen Formel

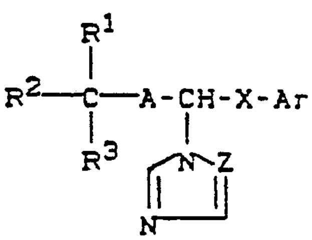

(I)

erhalten, die in der folgenden Tabelle formelmäßig aufgeführt sind:

| Bsp. Nr. | $R^1$ | $R^2$ | $R^3$ | A | Z | X | Ar | physikalische Daten |
|---|---|---|---|---|---|---|---|---|
| 7 | $CHCl_2$ | $CH_3$ | $CH_3$ | CHOH | CH | O | 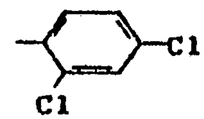 | Harz * |
| 8 | $CHCl_2$ | $CH_3$ | $CH_3$ | CO | N | O | 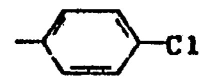 | Fp: 59-60° C |
| 9 | $CHCl_2$ | $CH_3$ | $CH_3$ | CHOH | N | O | 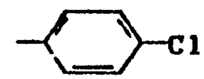 | Fp: 129-135° C |
| 10 | $CHCl_2$ | $CH_3$ | $CH_3$ | CO | CH | O | 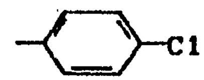 | $n_D^{25}$: 1,555 |
| 11 | $CHCl_2$ | $CH_3$ | $CH_3$ | CHOH | CH | O | 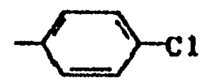 | Fp: 90-134° C |
| 12 | $CHCl_2$ | $CH_3$ | $CH_3$ | CO | N | O | 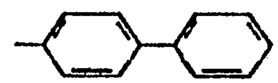 | Fp: 177-178° C |
| 13 | $CHCl_2$ | $CH_3$ | $CH_3$ | CO | CH | O | 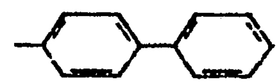 | Harz |

*) Diastereomerengemisch

| Bsp. Nr. | $R^1$ | $R^2$ | $R^3$ | A | Z | X | Ar | physikalische Daten |
|---|---|---|---|---|---|---|---|---|
| 14 | n-$C_4H_9$ | $C_2H_5$ | $CH_3$ | CO | N | O | 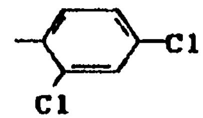 | Harz |
| 15 | n-$C_4H_9$ | $C_2H_5$ | $CH_3$ | CO | CH | O | 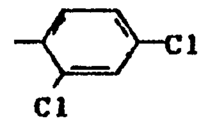 | Harz |
| 16 | n-$C_4H_9$ | $C_2H_5$ | $CH_3$ | CHOH | N | O | 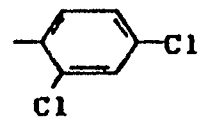 | Harz * |
| 17 | n-$C_4H_9$ | $C_2H_5$ | $CH_3$ | CHOH | CH | O | 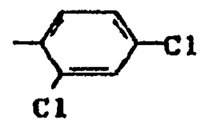 | Harz * |
| 18 | n-$C_4H_9$ | $C_2H_5$ | $CH_3$ | CO | N | O | 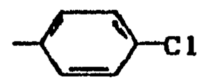 | Harz |
| 19 | n-$C_4H_9$ | $C_2H_5$ | $CH_3$ | CHOH | N | O | 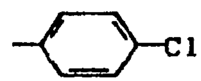 | Harz * |

*) Diastereomerengemisch

| Bsp. Nr. | $R^1$ | $R^2$ | $R^3$ | A | Z | X | Ar | physikalische Daten |
|---|---|---|---|---|---|---|---|---|
| 20 | n-$C_4H_9$ | $C_2H_5$ | $CH_3$ | CO | N | O | 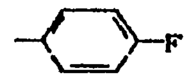 | Harz |
| 21 | n-$C_4H_9$ | $C_2H_5$ | $CH_3$ | CO | CH | O | 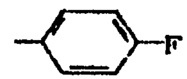 | Harz |
| 22 | n-$C_4H_9$ | $C_2H_5$ | $CH_3$ | CHOH | N | O | 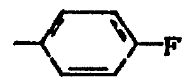 | Harz * |
| 23 | n-$C_4H_9$ | $C_2H_5$ | $CH_3$ | CHOH | CH | O | 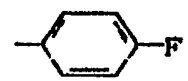 | Harz * |
| 24 | n-$C_4H_9$ | $C_2H_5$ | $CH_3$ | CO | CH | O | 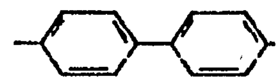 | Harz |
| 25 | n-$C_4H_9$ | $C_2H_5$ | $CH_3$ | CHOH | CH | O | 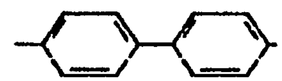 | visk. Öl * |
| 26 | $C_2H_5$ | $C_2H_5$ | $CH_3$ | CHOH | N | O | 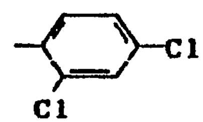 | visk. Öl * |

*) Diastereomerengemisch

| Bsp. Nr. | $R^1$ | $R^2$ | $R^3$ | A | Z | X | Ar | physikalische Daten |
|---|---|---|---|---|---|---|---|---|
| 27 | $C_2H_5$ | $C_2H_5$ | $CH_3$ | CO | CH | O | 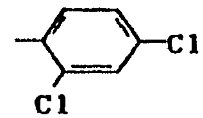 | visk. Öl |
| 28 | $C_2H_5$ | $C_2H_5$ | $CH_3$ | CHOH | CH | O | 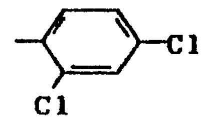 | visk. Öl |
| 29 | $C_2H_5$ | $C_2H_5$ | $CH_3$ | CO | N | O | 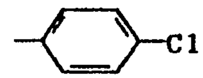 | visk. Öl |
| 30 | $C_2H_5$ | $C_2H_5$ | $CH_3$ | CO | CH | O | 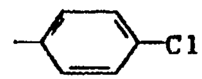 | visk. Öl |
| 31 | $C_2H_5$ | $C_2H_5$ | $CH_3$ | CHOH | N | O | 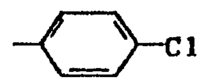 | visk. Öl |
| 32 | $C_2H_5$ | $C_2H_5$ | $CH_3$ | CHOH | CH | O | 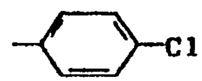 | Fp: 134-136° C* |
| 33 | n-$C_4H_9$ | $C_2H_5$ | $CH_3$ | CO | CH | O | 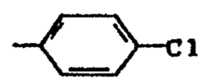 | Harz |

*) Diastereomerengemisch

| Bsp. Nr. | $R^1$ | $R^2$ | $R^3$ | A | Z | X | Ar | physikalische Daten |
|---|---|---|---|---|---|---|---|---|
| 34 | $CHCl_2$ | $CH_3$ | $CH_3$ | CHOH | N | O | 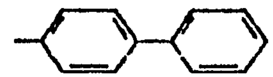 | Fp: 123-125° C |
| 35 | n-$C_4H_9$ | $C_2H_5$ | $CH_3$ | CHOH | CH | O | 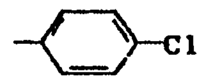 | Harz * |
| 36 | $CHCl_2$ | $CH_3$ | $CH_3$ | CHOH | N | O | 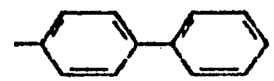 | Fp: 149-151° C reines Diastereomer |

* = Diastereomerengemisch

Verwendungsbeispiele:

In den nachfolgenden Verwendungsbeispielen werden die nachstehend angegebenen Substanzen als Vergleichssubstanz eingesetzt:

(A)

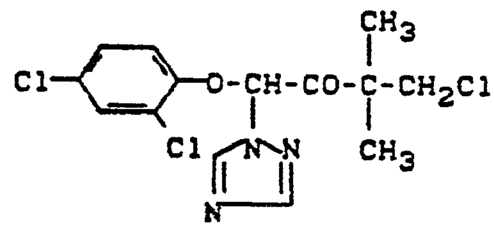

(B)

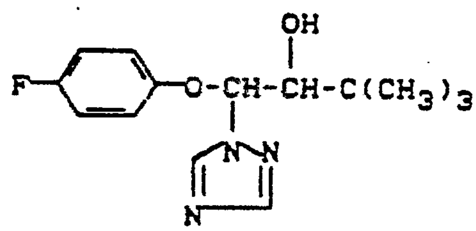

(C)

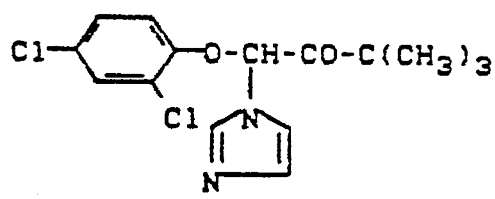

(D)

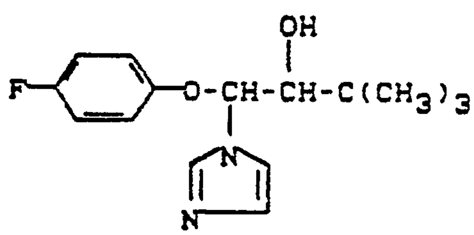

(E)

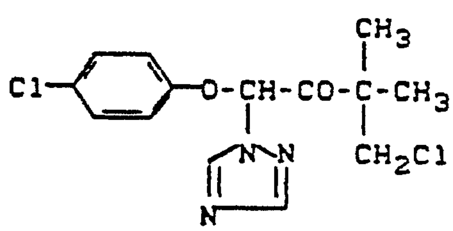

(F) 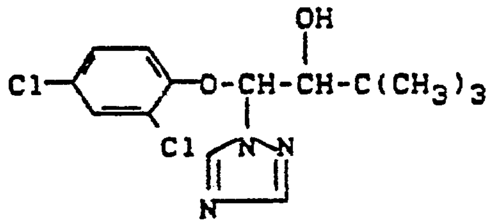

(G) 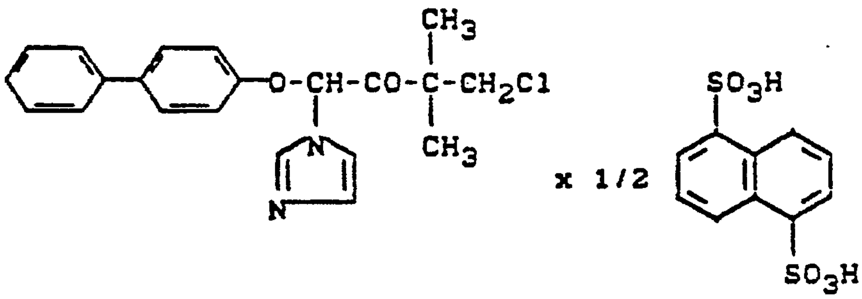

x 1/2

(H) 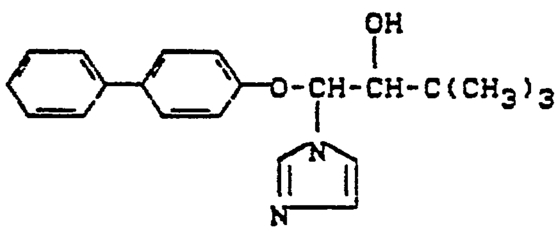

(J) 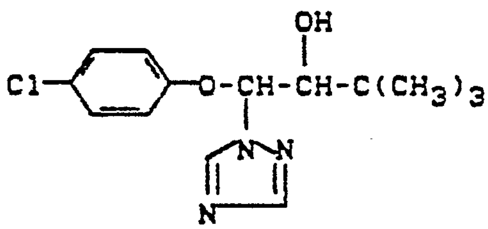

(K) 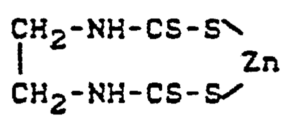

Beispiel A

Puccinia-Text (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Planzen mit einer Sporensuspension von Puccinia recondita mit in einer 0,1%igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt, um die Entwicklung von Rostpusteln zu begünstigen.

10 Tage nach der Inokulation erfolgt die Auswertung.

In diesem Test zeigen die erfindungsgemäßen Stoffe, die in den Beispielen 8 und 12 beschrieben sind, eine deutlich bessere Wirksamkeit als die Vergleichssubstanz (A).

Beispiel B

Leptosphaeria nodorum-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung.

In diesem Test zeigen die erfindungsgemäßen Stoffe, die in den Beispielen 19, 34 und 36 beschrieben sind, eine deutlich bessere Wirksamkeit als die Vergleichssubstanz (B). Ferner zeigen die erfindungsgemäßen Stoffe, die in den Beispielen 13, 15 und 33 beschrieben sind, eine deutlich bessere Wirksamkeit als die Vergleichssubstanz (C). Schließlich zeigt der erfindungsgemäße Stoff, der im Beispiel 17 beschrieben ist, eine deutlich bessere Wirksamkeit als die Vergleichssubstanz (D).

Beispiel C

Uromyces-Test (Buschbohne) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen das Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22°C und 100% relativer Luftfeuchtigkeit.

Die Pflanzen werden dann unter intensiver Belichtung für 9 Tage bei 20 bis 22°C und einer relativen Luftfeuchtigkeit von 70 bis 80% im Gewächshaus aufgestellt.

10 Tage nach der Inokulation erfolgt die Auswertung.

In diesem Test zeigen die erfindungsgemäßen Stoffe, die in den Beispielen 8 und 12 beschrieben sind, eine deutlich bessere Wirksamkeit als die Vergleichssubstanz (E). Ferner zeigen die erfindungsgemäßen Stoffe, die in den Beispielen 1, 9, 19, 31 und 34 beschrieben sind, eine deutlich bessere Wirksamkeit als die Vergleichssubstanz.

Beispiel D

Venturia-Test (Apfel) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigen die erfindungsgemäßen Stoffe, die in den Beispielen 15 und 21 beschrieben sind, eine deutlich bessere Wirksamkeit als die Vergleichssubstanz (G).

Beispiel E

Sphaerotheca-Text (Gurke) / protektiv
Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff

mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschliessend bei 23 bis 24°C und bei einer relativen Luftfeuchtigkeit von ca. 75% im Gewächshaus aufgestellt.

10 Tage nach der Inokulation erfolgt die Auswertung.

In diesem Test zeigen die erfindungsgemäßen Stoffe, die in den Beispielen 23, 25, 28, 32 und 35 beschrieben sind, eine deutlich bessere Wirksamkeit als die Vergleichssubstanz (H).

Beispiel F

Pyricularia-Test (Reis) / protektiv

Lösungsmittel: 12,5 Gewichtsteile Aceton

Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bie 100% rel. Luftfeuchtigkeit und 25°C aufgestellt.

4 Tage nach der Inokulation erfolgt die Auswertung des Krankheitsbefalls.

In diesem Test zeigen die erfindungsgemäßen Stoffe, die in den Beispielen 9, 16, 31 und 34 beschrieben sind, eine deutlich bessere Wirksamkeit als die Vergleichssubstanz (J).

Ferner zeigen die erfindungsgemäßen Stoffe, die in den Beispielen 13, 15, 21, 24, 30 und 33 beschrieben sind, eine deutlich bessere Wirksamkeit als die Vergleichssubstanz (K).

Beispiel G

Wachstum bei Baumwolle

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird bei allen Pflanzen der Zuwachs gemessen und in Prozent des Zuwachses der Kontrollpflanzen berechnet. Es bedeuten 100% einen Zuwachs wie bei den Kontrollen, Werte unter 100% geben eine Wuchshemmung, Werte über 100% eine Wuchsförderung an.

In diesem Test zeigt der erfindungsgemäße Stoff, der im Beispiel 16 beschrieben ist, eine sehr starke wuchshemmende Wirkung.

Beispiel H

Wachstum bei Zuckerrüben

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird bei allen Pflanzen der Zuwachs gemessen und in Prozent des Zuwachses der Kontrollpflanzen berechnet. Es bedeuten 100% einen Zuwachs wie bei den Kontrollen, Werte unter 100% geben eine Wuchshemmung, Werte über 100% eine Wuchsförderung wieder.

In diesem Test zeigen die erfindungsgemäßen Stoffe, die in den Beispielen 9 und 16 beschrieben sind, eine starke wuchshemmende Wirkung.

## Patentansprüche

1. Azolyletherketone und -alkohole der Formel

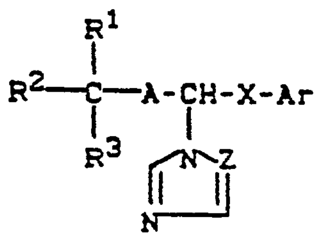

(I)

in welcher
$R_1$ für geradkettiges oder verzweigtes Alkyl mit 2 bis 4 Kohlenstoffatomen steht,
$R_2$ für geradkettiges oder verzweigtes Alkyl mit 2 bis 4 Kohlenstoffatomen steht,
$R_3$ für geradkettiges oder verzweigtes Alkyl mit 1 bis 4 Kohlenstoffatomen steht,
A für die Ketogruppe oder eine CH(OH)-Gruppe steht,
Z für ein Stickstoffatom oder die CH-Gruppe steht,
X für Sauerstoff steht und
Ar für Phenyl steht, das einfach bis dreifach, gleichartig oder verschieden substituiert sein kann durch Fluor, Chlor, Methyl und Phenyl,
oder
$R^1$ für Dichlormethyl steht,
$R^2$ für Methyl steht,
$R^3$ für Methyl steht,
A für die Ketograuppe oder eine CH(OH)-Gruppe steht,
Z für ein Stickstoffatom oder die CH-Gruppe steht,
X für Sauerstoff steht und
Ar für Phenyl steht, das einfach bis dreifach, gleichartig oder verschieden substituiert sein kann durch Fluor, Chlor, Methyl und Phenyl,
sowie deren Säureadditions-Salze und Metallsalz-Komplexe.

2. Verfahren zur Herstellung von Azolyletherketonen und -alkoholen der Formel

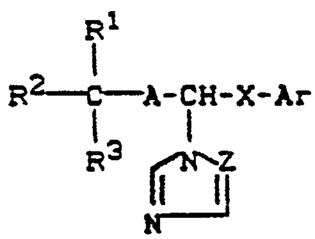

(I)

in welcher
R1 für geradkettiges oder verzweigtes Alkyl mit 2 bis 4 Kohlenstoffatomen steht,
$R^2$ für geradkettiges oder verzweigtes Alkyl mit 2 bis 4 Kohlenstoffatomen steht,
$R_3$ für geradkettiges oder verzweigtes Alkyl mit 1 bis 4 Kohlenstoffatomen steht,
A für die Ketogruppe oder eine CH(OH)-Gruppe steht,
Z für ein Stickstoffatom oder die CH-Gruppe steht,
X für Sauerstoff steht und
Ar für Phenyl steht, das einfach bis dreifach, gleichartig oder verschieden substituiert sein kann durch Fluor, Chlor, Methyl und Phenyl,
oder
$R^1$ für Dichlormethyl steht,
$R_2$ für Methyl steht,
$R_3$ für Methyl steht,
A für die Ketogruppe oder eine CH(OH)-Gruppe steht,
Z für ein Stickstoffatom oder die CH-Gruppe steht,
X für Sauerstoff steht und
Ar für Phenyl steht, das einfach bis dreifach, gleichartig oder verschieden substituiert sein kann durch Fluor, Chlor, Methyl und Phenyl,
sowie deren Säureadditionssalzen und Metallsalz-Komplexen, dadurch gekennzeichnet, daß man

a) Halogenetherketone der Formel

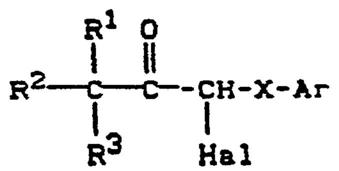

(II)

in welcher
$R^1$, $R^2$, $R^3$, X und Ar die oben angegebenen Bedeutungen haben und
Hal für Chlor oder Bron steht,
mit 1,2,4-Triazol oder Imidazol, gegebenenfalls in Gegenwart eines Säurebindemittels und gegebenenfalls in Gegenwart eines Verdünnungsmittels, umsetzt und gegebenenfalls
b) die nach Verfahren (a) erhaltenen Azolyletherketone der Formel

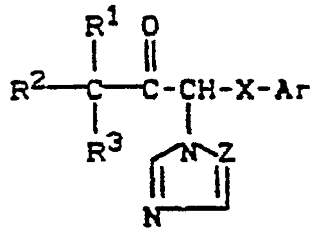

(Ia)

in welcher
$R^1$, $R^2$, $R^3$, Z, X und Ar die oben angegebenen Bedeutungen haben,
gegebenenfalls in Gegenwart eines Verdünnungsmittels reduziert,
und gegebenenfalls anschließend eine Säure oder ein Metallsalz addiert.

3. Fungizide und pflanzenwuchsregulierende Mittel, gekennzeichnet durch einen Gehalt an mindestens einem Azolyletherketon bzw. -alkohol der Formel (I) gemäß Anspruch 1 bzw. an einem Säureadditions-Salz oder Metallsalz-Komplex eines Azolyletherketons bzw. -alkohols der Formel (I).

4. Verwendung von Azolyletherketonen und -alkoholen der Formel (I) gemäß Anspruch 1 bzw. von deren Säureadditions-Salzen und Metallsalz-Komplexen zur Bekämpfung von Pilzen.

5. Verwendung von Azolyletherketonen und -alkoholen der Formel (I) gemäß Anspruch 1 bzw. von deren Säureadditionssalzen und Metallsalz-Komplexen zur Regulierung des Pflanzenwachstums.

6. Verfahren zur Bekämpfung von Pilzen, dadurch gekennzeichnet, daß man Azolyletherketone und -alkohole der Formel (I) gemäß Anspruch 1 bzw. deren Säureadditions-Salze oder Metallsalz-Komplexe auf Pilze und/oder deren Lebensraum ausbringt.

7. Verfahren zur Regulierung des Pflanzenwachstums, dadurch gekennzeichnet, daß man Azolyletherketone oder -alkohole der Formel (I) gemäß Anspruch 1 bzw. deren Säureadditionssalzen oder Metallsalz-Komplexe auf die Pflanzen und/oder deren Lebensraum ausbringt.

8. Verfahren zur Herstellung von fungiziden und pflanzenwuchsregulierenden Mitteln, dadurch gekennzeichnet, daß man Azolyletherketone oder -alkohole der Formel (I) gemäß Anspruch 1 bzw. deren Säureadditionssalze oder Metallsalz-Komplexe mit Streckmitteln und/oder oberflächenaktiven Stoffen vermischt.

9. Azolyletherketon gemäß Anspruch 1, gekennzeichnet durch die Formel

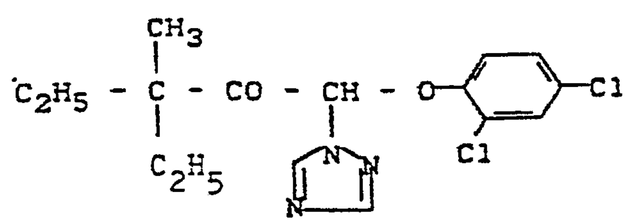

## Claims

1. Azolyl ether ketones and azolyl ether alcohols of the formula

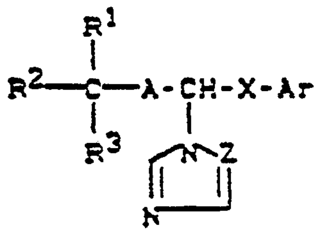

(I)

in which
$R^1$ represents straight-chain or branched alkyl with 2 to 4 carbon atoms,
$R^2$ represents straight-chain or branched alkyl with 2 to 4 carbon atoms,
$R^3$ represents straight-chain or branched alkyl with 1 to 4 carbon atoms,
A represents the keto group or a CH(OH) group,
Z represents a nitrogen atom or the CH group,
X represents oxygen and
Ar represents phenyl which is optionally monosubstituted to trisubstituted by identical or different substituents from amongst fluorine, chlorine, methyl and phenyl,
or $R^1$ represents dichloromethyl
$R^2$ represents methyl,
$R^3$ represents methyl,
A represents the keto group or a CH(OH) group,
Z represents a nitrogen atom or the CH group,
X represents oxygen and
Ar represents phenyl which is optionally monosubstituted to trisubstituted by identical or different substituents, from amongst fluorine, chlorine, methyl and phenyl, and acid addition salts and metal salt complexes thereof.

2. Process for the preparation of azolyl ether ketones and azolyl ether alcohols of the formula

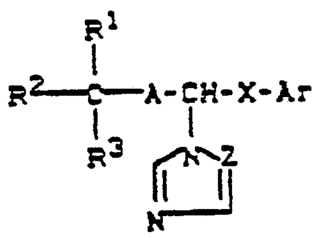

(I)

in which
$R^1$ represents straight-chain or branched alkyl with 2 to 4 carbon atoms,
$R^2$ represents straight-chain or branched alkyl with 2 to 4 carbon atoms,
$R^3$ represents straight-chain or branched alkyl with 1 to 4 carbon atoms,
A represents the keto group or a CH(OH) group,
Z represents a nitrogen atom or the CH group,
X represents oxygen and
Ar represents phenyl which is optionally monosubstituted to trisubstituted by identical or different substituents from amongst fluorine, chlorine, methyl and phenyl,
or $R_1$ represents dichloromethyl,
$R_2$ represents methyl,
$R_3$ represents methyl,
A represents the keto group or a CH(OH) group,
Z represents a nitrogen atom or the CH group,
X represents oxygen and
Ar represents phenyl which is optionally monosubstituted to trisubstituted by identical or different substituents, from amongst fluorine, chlorine, methyl and phenyl, and acid addition salts and metal salt complexes thereof, characterized in that

a) halogenoether ketones of the formula

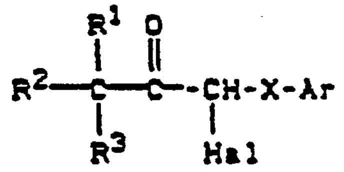

(II)

in which
$R_1$, $R^2$, $R^3$, X and Ar have the abovementioned meanings and
Hal represents chlorine or bromine,
are reacted with 1,2,4-triazole or imidazole, if appropriate in the presence of an acid-binding agent and if appropriate in the presence of a diluent, and, if appropriate,
b) the azolyl ether ketones obtained by process (a), of the formula

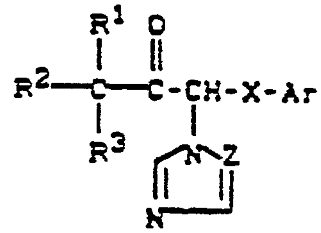

(Ia)

in which
$R^1$, $R^2$, $R^3$, Z, X and Ar have the abovementioned meanings,
are reduced, if appropriate in the presence of a diluent, and, if appropriate, an acid or a metal salt is then added on.

3. Fungicidal and plant growth-regulating agents, characterized in that they contain at least one azolyl ether ketone or azolyl ether alcohol of the formula (I) according to Claim 1 or an acid addition salt or metal salt complex of an azolyl ether ketone or of an azolyl ether alcohol of the formula (I).

4. Use of azolyl ether ketones and azolyl ether alcohols of the formula (I) according to Claim 1 or of acid addition salts and metal salt complexes thereof for combating fungi.

5. Use of azolyl ether ketones and azolyl ether alcohols of the formula (I) according to Claim 1 or of acid addition salts and metal salt complexes thereof, for regulating plant growth.

6. Method of combating fungi, characterized in that azolyl ether ketones and azolyl ether alcohols of the formula (I) according to Claim 1 or acid addition salts or metal salt complexes thereof are applied to fungi and/or their environment.

7. Method of regulating plant growth, characterized in that azolyl ether ketones or azolyl ether alcohols of the formula (I) according to Claim 1 or acid addition salts or metal salt complexes thereof are applied to the plants and/or their environment.

8. Process for the preparation of fungicidal and plant growth-regulating agents, characterized in that azolyl ether ketones or azolyl ether alcohols of the formula (I) according to Claim 1 or acid additions salts or metal salt complexes thereof are mixed with extenders and/or surface-active substances.

9. Azolyl ether ketone according to Claim 1, characterized by the formula

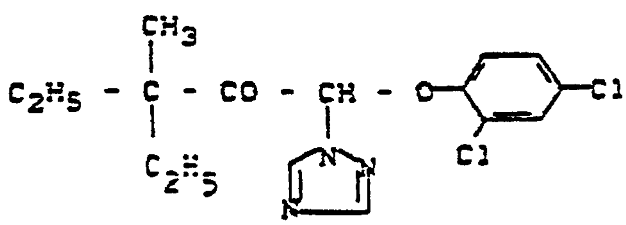

## Revendications

1. Azolyl-éther-alcools et -cétones de formule:

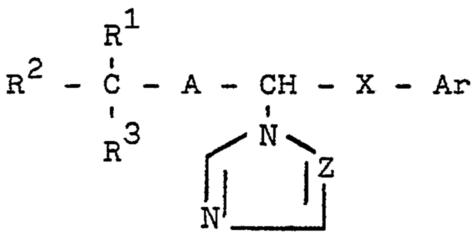

(I)

dans laquelle
$R^1$ représente un groupe alkyle à chaîne droite ou ramifiée contenant 2 à 4 atomes de carbone,
$R^2$ représente un groupe alkyle à chaîne droite ou ramifiée contenant 2 à 4 atomes de carbone,
$R^3$ représente un groupe alkyle à chaîne droite ou ramifiée contenant 1 à 4 atomes de carbone,
A représente le gropue céto ou un groupe CH(OH),
Z représente un atome d'azote ou le groupe CH,
X représente un atome d'oxygène, et
Ar représente un groupe phényle qui peut être substitué une à trois fois de manière identique ou différente par un atome de fluor, un atome de chlore, un groupe méthyle et un groupe phényle, ou
$R^1$ représente un groupe dichlorométhyle,
$R^2$ représente un groupe méthyle,
$R^3$ représente un groupe méthyle,
A représente le groupe céto ou un groupe CH(OH),
Z représente un atome d'azote ou le groupe CH,
X représente un atome d'oxygène, et
Ar représente un groupe phényle, qui peut être substituée une à trois foid de manière identique ou différente par un atome de fluor, un atome de chlore, un groupe méthyle et un groupe phényle,
ainsi que leurs sels d'addition d'acide et leurs complexes de sels métalliques.

2. Procédé de préparation d'azolyl-éther-alcools et -cétones de formule:

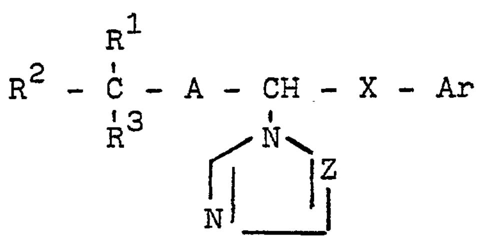

(I)

dans laquelle
$R^1$ représente un groupe alkyle à chaîne droite ou ramifiée contenant 2 à 4 atomes de carbone,
$R^2$ représente un groupe alkyle à chaîne droite ou ramifiée contenant 2 à 4 atomes de carbone,
$R^3$ représente un groupe alkyle à chaîne droite ou ramifiée contenant 1 à 4 atomes de carbone,
A représente le groupe céto ou un groupe CH(OH),
Z représente un atome d'azote ou le groupe CH,
X représente un atome d'oxygène, et
Ar représente un groupe phényle qui peut être substitué une à trois fois de manière identique ou différente par un atome de fluor, un atome de chlore, un groupe méthyle et un groupe phényle,
ou
$R^1$ représente un groupe dichlorométhyle,
$R^2$ représente un groupe méthyle,
$R^3$ représente un groupe méthyle,
A représente le groupe céto ou un groupe CH(OH),
Z représente un atome d'azote ou le groupe CH,
X représente un atome d'oxygène, et
Ar représente un groupe phényle qui peut être substitué une à trois fois de manière identique ou différente par un atome de fluor, un atome de chlore, un groupe méthyle et un groupe phényle,
ainsi que de leurs sels d'addition d'acide et leurs complexes de sels métalliques, caractérisé en ce que:

a) on fait réagir des halogéno-éther-cétones de formule:

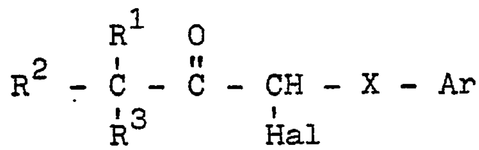 (II)

dans laquelle
$R^1$, $R^2$, $R^3$, X et Ar ont les significations indiquées ci-dessus, et
Hal représente un atome de chlore ou un atome de brome,
avec du 1 2,4-triazole ou de l'imidazole, éventuellement en présence d'un agent fixateur d'acide et éventuellement en présence d'un diluant, et éventuellement
b) on réduit les azolyl-éther-cétones obtenues selon le procédé (a) et répondant à la formule:

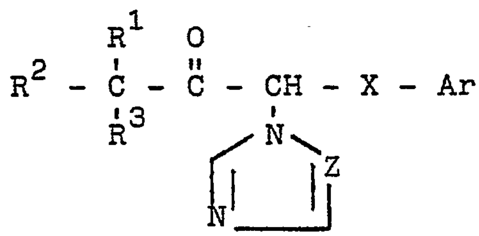 (Ia)

dans laquelle
$R^1$, $R^2$, $R^3$, Z, X et Ar ont les significations indiquées ci-dessus, éventuellement en présence d'un diluant, et on ajoute éventuellement ensuite un acide ou un sel de métal.

3. Agents fongicides et régulateurs de la croissance des plantes, carctérisés en ce qu'ils contiennent au moins un azolyl-éther-alcool ou -cétone de formule (I) selon la revendication 1 ou un sel d'addition d'acide ou encore un complexe de sels de métaux d'un azolyl-éther-alcool ou -cétone de formule (I).

4. Utilisation d'azolyl-éther-alcools ou -cétones de formule (I) selon la revendication 1 ou de leurs sels d'addition d'acide et de leurs complexes de sels de métaux en vue de combattre les champignons.

5. Utilisation d'azolyl-éther-alcools et -cétones de formule (I) selon la revendication 1 ou de leurs sels d'addition d'acide et de leurs complexes de sels de métaux pour la régulation de la croissance des plantes.

6. Procédé en vue de combattre les champignons, caractérisé en ce qu'on applique des azolyl-éther-alcools et -cétones de formule (I) selon la revendication 1, ou leurs sels d'addition d'acide ou encore leurs complexes de sels de métaux sur les champignons et/ou leur biotope.

7. Procédé pour la régulation de la croissance des plantes, caractérisé en ce qu'on applique des azolyl-éther-alcools ou -cétones de formule (I) selon la revendication 1 ou leurs sels d'addition d'acide ou encore leurs complexes de sels de métaux sur les plantes et/ou leur biotope.

8. Procédé de préparation d'agents fongicides et régulateurs de la croissance des plantes, caractérisé en ce qu'on mélange des azolyl-éther-alcools ou -cétones de formule (I) selon la revendication 1 ou leurs sels d'addition d'acide ou encore leurs complexes de sels de métaux avec des diluants et/ou des substances tensio-actives.

9. Azolyl-éther-cétone selon la revendication 1, caractérisée en ce qu'elle répond à la formule